(19) Europäisches Patentamt
European Patent Office
Office européen des brevets

(11) Numéro de publication: **0 102 896**
**A1**

(12) # DEMANDE DE BREVET EUROPEEN

(21) Numéro de dépôt: **83401698.2**

(51) Int. Cl.³: **C 07 D 501/24**

(22) Date de dépôt: **23.08.83**

(30) Priorité: **27.08.82 FR 8214700**

(43) Date de publication de la demande:
**14.03.84 Bulletin 84/11**

(84) Etats contractants désignés:
**AT BE CH DE FR GB IT LI LU NL SE**

(71) Demandeur: **RHONE-POULENC SANTE**
**Les Miroirs 18 Avenue d'Alsace**
**F-92400 Courbevoie Cedex(FR)**

(72) Inventeur: **Berger, Christian**
**26 chemin de la Forestière Tour no 3**
**F-69130 Ecully(FR)**

(72) Inventeur: **Farge, Daniel**
**30 rue des Pins Sylvestres**
**F-94320 Thiais(FR)**

(72) Inventeur: **Le Roy, Pierre**
**2 allée des Cerisiers**
**F-94320 Thiais(FR)**

(72) Inventeur: **Moutonnier, Claude**
**3 rue Auguste Rodin**
**F-92350 Le Plessis Robinson(FR)**

(74) Mandataire: **Pilard, Jacques et al,**
**RHONE-POULENC RECHERCHES Service Brevets**
**Pharma 25, Quai Paul Doumer**
**F-92408 Courbevoie Cedex(FR)**

(54) **Formes cristallisées de sels d'addition avec des acides, de l'((amino-2 thiazolyl-4)-2 méthoxyimino-2 acétamido)-7 carboxy-2((dioxo-5,6 formylméthyl-4 tetrahydro-1,4,5,6 triazine-1,2,4 yl-3) thio-2 vinyl)-3 oxo-8 thia-5 aza-1 bicyclo(4.2.0) octène-2 isomère syn, forme E et leur préparation.**

(57) Formes cristallisées de sels d'addition avec les acides trifluoracétique, méthanesulfonique ou chlorhydrique de l'[(amino-2 thiazolyl-4)-2 méthoxyimino-2 acétamido]-7 carboxy-2 [(dioxo-5,6 formylméthyl-4 tetrahydro-1,4,5,6 triazine-1,2,4 yl-3) thio-2 vinyl]-3 oxo-8 thia-5 aza-1 bicyclo[4.2.0] octène-2, isomère syn forme E de formule:

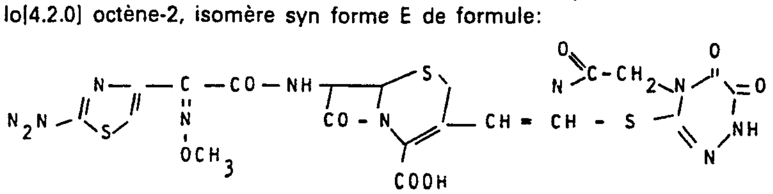

et leur préparation.

Ces sels sont utiles pour la purification du produit ci-dessus ainsi que pour l'utilisation en pharmacie

EP 0 102 896 A1

Croydon Printing Company Ltd

La présente invention a pour objet les formes cristallisées de sels d'addition avec les acides trifluoracétique, méthanesulfonique ou chlorhydrique de l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo [4.2.0] octène-2 isomère syn forme E et leur préparation.

Dans le brevet américain 4 307 116 a été décrit l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2 isomère syn, forme E, ci-après appelé produit (I) et ses sels, qui sont particulièrement intéressants comme agents anti-microbiens sur les germes gram-positifs ou gram-négatifs.

Le brevet américain 4 307 116 mentionne l'existence du substituant formylméthyle du produit (I), à l'état d'aldéhyde libre ou d'hydrate d'aldéhyde, et l'observation de ces formes dans les conditions rappelées ci-dessous :

les études de résonance magnétique nucléaire montrent en effet que :

- en solvant acide, tel que l'acide formique ou trifluoroacétique (deutérés), en présence ou non d'eau (lourde) le produit se présente principalement sous la forme d'aldéhyde libre.
- en solvant basique tel que l'eau (lourde) additionnée de bicarbonate de sodium, il se présente principalement sous la forme d'hydrate d'aldéhyde.
- en solvant neutre tel que diméthylsulfoxyde ($d_6$), les formes aldéhyde libre et hydrate d'aldéhyde sont présentes, l'addition d'eau entraînant progressivement la conversion de la forme aldéhyde libre en forme hydrate d'aldéhyde.

Selon les procédés décrits antérieurement, les sels du produit (I) ont toujours été obtenus sous forme amorphe, et de plus les opérations d'isolement et de purification du produit (I) ne permettaient pas obligatoirement de préparer des lots de même

degré de pureté.

Les sels selon l'invention peuvent être obtenus par cristallisation en milieu acide, en traitant le produit (I), un de ses sels, ou un solvat formique du produit (I), par un excès d'acide trifluoracétique,méthanesulfonique ou chlorhydrique.

Lorsque le produit (I) mis en oeuvre est utilisé à l'état de sel, celui-ci peut être choisi parmi les sels métalliques, les sels d'addition avec une base azotée et les sels d'addition avec un acide. Ces sels peuvent être obtenus après transformation du produit (I) par les méthodes habituelles, éventuellement sans isolement préalable à la cristallisation. Il est également avantageux d'utiliser directement les sels (ou le solvat formique) obtenus à l'issue de la synthèse du produit (I), après élimination des groupements protecteurs nécessités par la synthèse.

Par exemple on utilise les sels de sodium, de lithium ou le trifluoracétate du produit (I).

L'acidification, par l'acide trifluoracétique, méthanesulfonique ou chlorhydrique, s'effectue en présence d'un excès d'acide compris entre 2 et 300 moles par mole de produit (I) et à une température comprise entre 0 et 80°C. Le cas échéant, il est nécessaire de refroidir ultérieurement le mélange réactionnel à une température comprise entre -10 et 30°C,de manière à améliorer le rendement de la cristallisation.

A l'issue de l'acidification, la phase de cristallisation doit être claire et limpide. Le cas échéant il peut être nécessaire de filtrer le mélange préalablement à la cristallisation.

La cristallisation s'effectue en milieu aqueux ou hydroorganique.

Lorsque l'on cristallise les sels selon l'invention en milieu aqueux, il est avantageux de traiter un sel métallique ou un sel de base azotée du produit (I). Dans ce cas, le sel est choisi de telle manière qu'il conduise à une solution aqueuse en contenant au moins 0,2 %.

Lorsque l'on cristallise les sels selon l'invention en milieu hydroorganique, les solvants organiques sont choisis parmi les solvants miscibles à l'eau, par exemple les alcools (méthanol, éthanol, n.propanol, isopropanol), les cétones (acétone), le diméthylformamide, l'acétonitrile ou l'acide formique, ou encore des mélanges de ces solvants.

Les proportions du mélange hydroorganique peuvent varier largement, la quantité de solvant organique pouvant aller de 0 à 90 % du volume du mélange. On utilise avantageusement des mélanges 50/50 en volumes.

Lorsque la cristallisation est effectuée en milieu aqueux ou hydroorganique autre qu'hydroalcoolique, en accord avec les précisions du brevet américain 4 307 116, le radical formylméthyle du produit cristallisé peut exister sous forme d'aldéhyde libre ou d'hydrate d'aldéhyde, ou de leurs mélanges.

Lorsque la cristallisation est effectuée en milieu hydroalcoolique, le radical formylméthyle du produit cristallisé peut exister à l'état d'hémiacétal, ou à l'état de mélange de la forme hémiacétal et de la forme aldéhyde libre et éventuellement de forme la/ hydrate d'aldéhyde (la fonction aldéhyde du produit (I) en présence de l'alcool du milieu de cristallisation se trouvant en équilibre avec la fonction hémiacétal correspondante).

Les études de résonance magnétique nucléaire des sels cristallisés selon l'invention montrent dans ce cas :

- en solvant acide [acide formique ou trifluoracétique (deutérés)] en présence ou non d'eau (lourde), que le produit se présente principalement sous la forme d'aldéhyde libre ;
- en solvant neutre [diméthylsulfoxyde ($d_6$)] que les formes aldéhyde libre, hydrate d'aldéhyde et hémiacétal sont présentes, l'addition d'eau entraînant progressivement la conversion de ces formes en forme hydrate d'aldéhyde.

Il est entendu que les sels cristallisés d'addition avec les acides trifluoracétique, méthanesulfonique ou chlorhydrique du produit (I) dont le radical formylméthyle se présente sous l'une des formes citées ci-dessus, ou leurs mélanges, entrent dans le cadre de la présente invention.

Les sels cristallisés, selon l'invention, obtenus en

totalité ou en partie à l'état d'hémiacétal, peuvent être convertis en sels du même acide, cristallisés, dont le substituant formylméthyle se trouve à l'état d'aldéhyde libre ou d'hydrate d'aldéhyde (ou leur mélange). On opère par mise en suspension du produit dans une solution aqueuse de l'acide correspondant.

Le produit (I), ses sels ou le solvat formique du produit (I) peuvent être préparés selon l'un des procédés décrits dans le brevet américain 4 307 116.

Les formes cristallisées des sels du produit (I) selon l'invention sont caractérisées par les propriétés physiques ci-dessous ou données ci-après dans les exemples :

- Aspect : solide blanc à blanc jaune
- Diagramme de diffraction des poudres aux rayons X [rayonnement CuKα avec filtre de nickel à une longueur d'onde λ = 1,54 Å].

Dans ces diagrammes (donnés ci-après dans les exemples) d représente la distance des plans inter-réticulaires en Å et $I/I_1$ représente les intensités relatives.

- Spectre infra-rouge (détermination à partir de comprimés en mélange avec KBr).

Ces spectres sont représentés par les figures 1 à 9 sur lesquelles on a porté en abscisses, d'une part les longueurs d'ondes exprimées en microns (échelle inférieure) et d'autre part les nombres d'ondes exprimés en $cm^{-1}$ (échelle supérieure) et en ordonnées les densités optiques.

Dans les tableaux (donnés ci-après à la suite de chaque exemple) sont indiquées les principales bandes d'absorption infra-rouge des sels cristallisés selon l'invention, exprimées en nombre d'ondes ($cm^{-1}$).

| | | |
|---|---|---|
| tF = très forte | m = moyenne | tf = très faible |
| F = forte | f = faible | ép = épaulement |

Les sels cristallisés selon l'invention sont hygroscopiques et peuvent contenir une teneur en eau pouvant aller jusqu'à 12 %.

La présente invention est particulièrement intéressante puisqu'elle permet d'une part d'obtenir des sels cristallisés du produit (I) mais aussi parce que cela constitue de ce fait une méthode de purification du produit (I) conduisant notamment à des lots purifiés de leurs impuretés.

Les formes cristallines selon l'invention présentent de plus une qualité supérieure du point de vue de leur stabilité.

Les sels cristallisés selon l'invention possèdent donc une valeur certaine, soit comme forme purifiée du produit (I), soit comme produit actif, dans un médicament, tel quel ou après traitement par une base compatible et pharmaceutiquement acceptable.

D'un intérêt particulier sont les formes cristallisées de sels d'addition avec les acides trifluoracétique ou méthanesulfonique de l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2 dont le radical formylméthyle se présente à l'état de mélange des formes hémiacétal éthylique et aldéhyde libre ou des formes hémiacétal éthylique, hydrate d'aldéhyde et aldéhyde libre, ou encore des formes hydrate d'aldéhyde et aldéhyde libre.

Spécialement intéressants sont aussi les produits suivants :

- forme cristallisée du sel d'addition avec l'acide trifluoracétique de l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3)thio-2 vinyl]-3 oxo-8 thia-5 aza-1 bicyclo[4.2.0] octène-2 dont le radical formylméthyle se présente à l'état d'aldéhyde libre, d'hydrate d'aldéhyde ou d'hémiacétal ou du mélange de ces formes
- forme cristallisée du sel d'addition avec l'acide méthanesulfonique de l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3)thio-2 vinyl]-3 oxo-8 thia-5 aza-1 bicyclo[4.2.0] octène-2 dont le radical formylméthyle se présente à l'état d'aldéhyde libre, d'hydrate d'aldéhyde ou d'hémiacétal ou du mélange de ces formes
- forme cristallisée du sel d'addition avec l'acide chlorhydrique de l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2 dont le radical formylméthyle se présente à l'état d'aldéhyde libre, d'hydrate d'aldéhyde ou d'hémiacétal ou du mélange de ces formes.

Les exemples suivants donnés à titre non limitatif illustrent la présente invention.

EXEMPLE 1

On dissout 5,6 g de trifluoracétate de l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2 isomère syn forme E amorphe dans un mélange préalablement porté au reflux, de 50 cm3 d'eau distillée, 50 cm3 de méthanol et 2 cm3 d'acide trifluoracétique. On traite avec 0,2 g de noir décolorant et filtre à chaud. Un produit cristallise rapidement. On complète la cristallisation en refroidissant pendant 3 heures à 4°C.

On sépare les cristaux par filtration et on les lave avec 20 cm3 du mélange solvant de cristallisation à 4°C, puis avec 30 cm3 d'éther et sèche sous pression réduite (0,05 mm de mercure ; 6,7 Pa) à 20°C pendant 16 heures. On obtient ainsi 4,3 g du trifluoracétate de l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo [4.2.0] octène-2 isomère syn forme E, hémiacétal méthylique, sous forme de cristaux blancs.

Spectre de RMN du proton (250 MHz, $CF_3COOD$, δ en ppm, J en Hz) 3,89 (s, 2H, $-SCH_2-$) ; 4,31 (s, 3H, $-OCH_3$) ; 5,20 (s, 2H, $>N-CH_2-$) ; 5,33 (d, J = 5, 1H, H en 6) ; 6,05 (d, J = 5, 1H, H en 7) ; 7,25 et 7,76 (2d, J = 16, 2H, -CH=CH-) ; 7,5 (s, 1H, H du thiazole) ; 9,76 (s, 1H, -CHO ).

Spectre de RMN du proton (250 MHz, DMSO $d_6$, δ en ppm, J en Hz) - hémiacétal méthylique (60 %) : bandes caractéristiques 3,23 (s, 1,8 H, $-CH(OH)OC\underline{H}_3$) ; 4,71 (t, J = 5, 0,6 H, $-CH_2C\underline{H}(OH)OCH_3$) ;

12,68 (s, 0,6 H, -NH- de la triazine).

- forme aldéhyde (30 %) : bandes caractéristiques 9,56 (s, 0,3 H, -CHO) ; 12,80 (s, 0,3 H, -NH- de la triazine).

- forme hydrate (10 %) : bandes caractéristiques 5,03 (t, J = 5, 0,1 H, $-CH_2\underline{CH}(OH)_2$ ; 12,66 (s, 0,1 H, -NH- de la triazine).

Diagramme de diffraction des poudres aux rayons X /rayonnement CuKα avec filtre de nickel, λ = 1,54 Å/

| d (Å) | 11,33 | 8,34 | 4,51 | 7,08 | 5,87 | 3,74 | 3,87 | 6,51 | 2,76 | 3,59 |
|---|---|---|---|---|---|---|---|---|---|---|
| $I/I_1$ | 1 | 0,93 | 0,87 | 0,67 | 0,54 | 0,36 | 0,34 | 0,30 | 0,26 | 0,21 |

Spectre infra-rouge (KBr), bandes caractéristiques ($cm^{-1}$) (figure 1).

| | | | |
|---|---|---|---|
| 3350 ép (dont $H_2O$) | 1665 F | 1200 F | 780 ép |
| 3280 F | 1630 F | 1190 F | 770 m |
| 3220 m | 1590 m | 1185 ép | 750 ép |
| 3150 ép | 1555 ép | 1145 ép | 735 m |
| 3070 f | 1550 F | 1135 F | 720 m |
| 3000 tf | 1535 ép | 1115 ép | 710 ép |
| 2965 tf | 1490 f | 1070 ép | 700 ép |
| 2940 f | 1450 ép | 1055 F | 665 ép |
| 2890 tf | 1440 m | 995 m | 640 m |
| 2830 ép | 1425 ép | 970 f | 630 ép |
| 2780 ép | 1410 m | 955 f | 625 ép |
| 2620 ép | 1380 ép | 935 m | 600 tf |
| 2000 ép | 1360 F | 890 ép | 560 f |
| 1935 ép | 1315 ép | 870 tf | 515 f |
| 1780 F | 1305 m | 850 m | 485 ép |
| 1765 F | 1260 m | 840 ép | 455 tf |
| 1720 tF | 1250 ép | 820 tf | 430 f |
| 1685 F | 1220 ép | 800 m | 395 tf |
| | | | 365 ép |

Le trifluoracétate de l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2 isomère syn forme E utilisé comme produit de départ peut être obtenu de la manière suivante :

A une solution refroidie à 4°C de 20 g de benzhydryloxycarbonyl-2 {[(diméthoxy-2,2 éthyl)-4 dioxo-5,6 tétrahydro-1,4,5,6 triazine-1,2,4 yl-3] thio-2 vinyl}-3 [méthoxyimino-2 (tritylamino-2 thiazolyl-4)-2 acétamido]-7 oxo-8 thia-5 aza-1 bicyclo[4.2.0] octène-2, isomère syn, forme E dans 35 cm3 d'anisole, on ajoute en 15 minutes un mélange de 3,5 cm3 d'eau et 35 cm3 d'acide trifluoracétique. On enlève le bain réfrigérant, agite pendant 16 heures à 20°C, refroidit à nouveau à 4°C et ajoute rapidement 300 cm3 d'acétone. On filtre, lave le précipité par 5 fois 20 cm3 d'acétone, 5 fois 20 cm3 d'éther et sèche sous pression réduite (0,05 mm de mercure ; 6,7 Pa) à 20°C pendant 16 heures. On recueille 10,7 g de poudre jaune constituée d'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2, isomère syn forme E à l'état de trifluoracétate amorphe.

EXEMPLE 2

A 25 cm3 d'un mélange eau-éthanol-acide trifluoracétique 70-30-2 (en volumes) porté à ébullition, on ajoute 1 g de trifluoracétate de l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2 isomère syn, forme E amorphe, et 0,05 g de noir décolorant. On filtre à chaud et laisse la solution jaune clair se refroidir à 20°C. Le filtrat cristallise. On laisse encore 2 heures à 4°C puis essore le précipité, lave deux fois par 10 cm3 d'éthanol puis par 10 cm3 d'éther et sèche sous pression réduite pendant 16 heures à 20°C (0,05 mm de mercure ; 6,7 Pa). On obtient ainsi 0,81 g de trifluoracétate cristallisé d'[(amino-2 thiazolyl-4)-2

méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2 isomère syn, forme E hémiacétal éthylique.

Spectre de RMN du proton (250 MHz, DMSO $d_6$, δ en ppm, J en Hz)
- hémiacétal éthylique (86 %) : bandes caractéristiques 1,07 (t, J = 7, 2,58 H, $-CH_2-CH\genfrac{}{}{0pt}{}{OH}{OCH_2-\underline{CH_3}}$) ; 4,79 (t, J = 5, 0,86 H, $-CH_2-\underline{CH}\genfrac{}{}{0pt}{}{OH}{OC_2H_5}$) ; 12,66 (s, 0,86 H, -NH- de la triazine).
- forme aldéhyde (14 %) : bandes caractéristiques 9,56 (s, 0,14 H, -$\underline{CH}$O ) ; 12,79 (s, 0,14 H, -NH- de la triazine).

Diagramme de diffraction des poudres aux rayons X [rayonnement CuKα avec filtre de nickel, λ = 1,54 Å]

| d (Å) | 12,45 | 3,99 | 4,74 | 4,62 | 8,84 | 3,90 | 6,11 | 3,19 | 3,60 | 4,39 |
|---|---|---|---|---|---|---|---|---|---|---|
| $I/I_1$ | 1 | 0,64 | 0,63 | 0,58 | 0,54 | 0,54 | 0,45 | 0,45 | 0,42 | 0,42 |

Spectre infra-rouge (KBr), bandes caractéristiques ($cm^{-1}$) (figure 2).

| | | | | |
|---|---|---|---|---|
| 3420 ép | 1783 F | 1308 m | 855 m | 560 f |
| 3370 F | 1770 F | 1265 m | 840 tf | 518 f |
| 3290 m | 1722 tF | 1250 ép | 820 tf | 485 ép |
| 3220 m | 1686 tF | 1225 ép | 801 m | 455 tf |
| 3150 tf | 1668 F | 1203 ép | 780 ép | 440 ép |
| 3080 f | 1634 F | 1192 F | 770 m | 430 tf |
| 2980 f | 1590 m | 1155 ép | 755 ép | 397 tf |
| 2945 tf | 1560 m | 1140 m | 738 m | 360 ép |
| 2900 tf | 1550 m | 1115 f | 724 m | |
| 2830 ép | 1490 m | 1060 F | 710 tf | |
| 2780 ép | 1440 m | 997 m | 700 ép | |
| 2720 ép | 1430 ép | 971 tf | 670 ép | |
| 2600 ép | 1413 m | 958 tf | 645 m | |
| 2420 ép | 1380 ép | 941 m | 635 tf | |
| 2000 ép | 1365 F | 890 ép | 600 tf | |
| 1950 ép | 1315 ép | 875 tf | 570 ép | |

EXEMPLE 3

On met en suspension 3,07 g de trifluoracétate de l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2 isomère syn forme E amorphe dans 410 cm3 d'eau et ajoute, sous agitation, 0,728 g de bicarbonate de sodium puis laisse 15 minutes sous agitation. La solution jaune obtenue est filtrée sur un buchner garni d'une plaque filtrante de cellulose SAM 30 (provenant de la Société LA ROCHETTE, SEMPA, FRANCE). Le filtrat obtenu est versé rapidement et sous agitation dans un mélange de 88 cm3 d'acide trifluoracétique et 44 cm3 d'eau. La suspension formée est filtrée rapidement sur un verre fritté de porosité 3 garni d'un adjuvant de filtration neutre (terre d'infusoires). Le filtrat jaune clair est laissé 1 heure 15 minutes à 20°C puis 2 heures à 4°C. La suspension est filtrée, les cristaux blancs obtenus sont lavés 4 fois par 8 cm3 d'acétone et séchés sous pression réduite (0,05 mm de mercure; 6,7 Pa) à 20°C pendant 16 heures. On obtient ainsi 0,92 g du trifluoracétate cristallisé de l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2, isomère syn forme E.

Spectre de RMN du proton (250 MHz, DMSO $d_6$, δ en ppm, J en Hz)

- forme aldéhyde (75 %) : bandes caractéristiques 9,56 (s, 0,75 H, -CHO) ; 12,80 (s, 0,75 H, -NH- de la triazine.
- forme hydrate (25 %) : bandes caractéristiques 5,03 (t, J = 5, 0,25 H, $-CH_2\underline{CH}(OH)_2$) ; 12,64 (s, 0,25 H, -NH- de la triazine).

Diagramme de diffraction des poudres aux rayons X [rayonnement CuKα avec filtre de nickel, λ = 1,54 Å]

| d (Å) | 4,13 | 10,53 | 14,06 | 4,25 | 3,95 | 6,86 | 14,73 | 4,44 | 7,69 | 7,37 |
|---|---|---|---|---|---|---|---|---|---|---|
| $I/I_1$ | 1 | 0,64 | 0,59 | 0,57 | 0,47 | 0,45 | 0,44 | 0,43 | 0,40 | 0,35 |

Spectre infra-rouge (KBr), bandes caractéristiques ($cm^{-1}$) (figure 3).

| | | | | |
|---|---|---|---|---|
| 3430 ép ($H_2O$) | 2000 ép | 1290 tf | 965 ép | 670 tf |
| 3380 F | 1775 F | 1260 m | 950 m | 630 m |
| 3320 m | 1720 F | 1250 ép | 920 tf | 625 ép |
| 3240 ép | 1695 ép | 1220 ép | 885 ép | 600 f |
| 3090 ép | 1680 tF | 1205 F | 875 f | 570 f |
| 3070 f | 1670 ép | 1185 F | 850 m | 555 tf |
| 3030 ép | 1630 ép | 1165 ép | 840 m | 540 ép |
| 2950 tf | 1595 F | 1140 tf | 820 tf | 515 f |
| 2900 tf | 1550 F | 1130 tf | 800 m | 470 ép |
| 2880 ép | 1495 tf | 1120 ép | 785 ép | 450 ép |
| 2830 ép | 1430 m | 1095 m | 765 f | 435 tf |
| 2740 ép | 1375 ép | 1060 ép | 745 m | 410 f |
| 2690 ép | 1360 F | 1050 F | 720 m | 390 ép |
| 2600 ép | 1325 ép | 1020 ép | 710 ép | 350 tf |
| 2420 ép | 1310 f | 995 m | | |

EXEMPLE 4

On ajoute 2 g de trifluoracétate de l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2, isomère syn, forme E amorphe dans un mélange de 75 cm3 d'eau distillée et 75 cm3 de méthanol. On porte à 70-75°C pendant 15 minutes. On sépare un insoluble par filtration. On refroidit à 40°C puis on ajoute 10 cm3 d'une solution aqueuse à 5 % d'acide méthanesulfonique et on laisse refroidir à 20°C. On laisse une nuit à 4°C.

On sépare les cristaux par filtration et on les lave avec 45 cm3 d'une solution de lavage préparée avec 50 cm3 d'eau, 50 cm3 de méthanol et 2 cm3 d'acide méthanesulfonique puis on les sèche sous pression réduite (0,05 mm de mercure ; 6,7 Pa) à 20°C pendant 16 heures. On obtient ainsi 1,34 g du méthanesulfonate de l'[(amino-2 thiazolyl-4)-2 méthoxyimino-2 acétamido]-7 carboxy-2 [(dioxo-5,6

formylméthyl-4 tétrahydro-1,4,5,6 triazine-1,2,4 yl-3) thio-2 vinyl]-3 oxo-8 thia-5 aza-1 bicyclo[4.2.0] octène-2, isomère syn forme E, hémiacétal méthylique, sous forme de cristaux blanc jaune.

Spectre de RMN du proton (250 MHz, DMSO $d_6$, δ en ppm, J en Hz)

- hémiacétal méthylique (50 %) : bandes caractéristiques 3,23 (s, 1,5 H, $-CH(OH)OC\underline{H}_3$) ; 4,71 (t, J = 5, 0,5 H, $-CH_2C\underline{H}(OH)OCH_3$) ; 12,68 (s, 0,5 H, -NH- de la triazine).
- forme aldéhyde (40 %) : bandes caractéristiques 9,56 (s, 0,4 H, $C\underline{H}O$) ; 12,80 (s, 0,4 H, -NH- de la triazine).
- forme hydrate (10 %) : bandes caractéristiques 5,03 (t, J = 5, 0,1 H, $-CH_2C\underline{H}(OH)_2$) ; 12,66 (s, 0,1 H, -NH- de la triazine).

Diagramme de diffraction des poudres aux rayons X [rayonnement CuKα avec filtre de nickel, λ = 1,54 Å]

| d (Å) | 11,63 | 5,64 | 8,27 | 4,31 | 4,39 | 3,41 | 7,25 | 3,87 | 3,74 | 3,62 |
|---|---|---|---|---|---|---|---|---|---|---|
| $I/I_1$ | 1 | 0,64 | 0,56 | 0,50 | 0,43 | 0,42 | 0,40 | 0,37 | 0,37 | 0,33 |

Spectre infra-rouge (KBr), bandes caractéristiques ($cm^{-1}$) (figure 4).

| | | | | |
|---|---|---|---|---|
| 3350 ép ($H_2O$) | 2000 ép | 1450 ép | 1040 F | 705 ép |
| 3310 ép | 1770 tF | 1440 ép | 1000 m | 670 tf |
| 3290 F | 1720 tF | 1420 ép | 965 ép | 645 ép |
| 3220 tf | 1710 ép | 1360 F | 945 m | 640 m |
| 3160 ép | 1675 F | 1315 m | 890 ép | 605 ép |
| 3070 ép | 1660 ép | 1255 ép | 855 m | 555 m |
| 3040 ép | 1635 F | 1250 tf | 820 ép | 525 m |
| 2940 f | 1585 F | 1205 m | 800 f | 480 ép |
| 2900 tf | 1555 ép | 1180 F | 780 m | 425 tf |
| 2840 ép | 1545 m | 1155 m | 740 m | 395 f |
| 2780 ép | 1530 ép | 1110 m | 715 tf | 360 ép |
| 2620 ép | 1470 ép | | | |

EXEMPLE 5

On porte à reflux 250 cm3 d'un mélange eau-éthanol-acide méthanesulfonique 50-50-0,2 (en volumes). On ajoute alors 5 g de méthanesulfonate de l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2, isomère syn, forme E qui se dissout immédiatement. Le mélange est laissé refroidir jusqu'à 20°C, puis placé 3 heures à 0°C. Les cristaux sont filtrés, lavés 3 fois par 5 cm3 du solvant de cristallisation, 2 fois par 10 cm3 d'éthanol et enfin par 5 cm3 d'éther, puis séchés sous pression réduite (0,05 mm de mercure ; 6,7 Pa) à 20°C pendant 16 heures. On obtient ainsi 4,3 g de méthanesulfonate de l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2, isomère syn, forme E, hémiacétal éthylique cristallisé.

Spectre de RMN du proton (250 MHz, DMSO $d_6$, δ en ppm, J en Hz)
- hémiacétal éthylique (75 %) : bandes caractéristiques 1,07 (t, J = 7, 2,25 H, $-CH_2CH{<}^{OH}_{OCH_2\underline{CH_3}}$) ; 4,79 (t, J = 5, 0,75 H, $-CH_2\underline{CH}{<}^{OH}_{OC_2H_5}$) ; 12,66 (s, 0,75 H, -NH- de la triazine).
- forme aldéhyde (25 %) : bandes caractéristiques 9,56 (s, 0,25 H, -C$\underline{H}$O) ; 12,80 (s, 0,25 H, -NH- de la triazine).

Diagramme de diffraction des poudres aux rayons X [rayonnement CuKα avec filtre de nickel, λ = 1,54 Å]

| d (Å) | 3,88 | 4,62 | 3,41 | 3,80 | 3,75 | 3,53 | 9,02 | 4,35 | 3,19 | 3,13 |
|---|---|---|---|---|---|---|---|---|---|---|
| $I/I_1$ | 1 | 0,84 | 0,84 | 0,79 | 0,63 | 0,63 | 0,60 | 0,55 | 0,54 | 0,51 |

Spectre infra-rouge (KBr), bandes caractéristiques ($cm^{-1}$) (figure 5).

| | | | | |
|---|---|---|---|---|
| 3400 F | 1770 F | 1415 ép | 1000 m | 670 ép |
| 3285 m | 1718 tF | 1365 F | 965 ép | 635 m |
| 3220 ép | 1675 F | 1315 m | 955 ép | 600 ép |
| 3070 tf | 1665 ép | 1262 f | 940 m | 555 m |
| 3020 ép | 1635 m | 1245 ép | 890 ép | 530 m |
| 2975 f | 1585 F | 1220 ép | 855 m | 490 ép |
| 2940 f | 1560 F | 1200 F | 820 ép | 455 ép |
| 2900 f | 1550 m | 1180 f | 800 f | 425 tf |
| 2825 ép | 1475 ép | 1160 m | 780 m | 400 f |
| 2740 ép | 1460 ép | 1110 m | 738 m | 375 ép |
| 2580 ép | 1440 f | 1045 F | 707 tf | 350 ép |
| 1990 ép | | | | |

EXEMPLE 6

On traite 10 g de trifluoracétate de l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2, isomère syn forme E amorphe en suspension dans 1 litre d'eau par 2,36 g de bicarbonate de sodium. Après 10 minutes d'agitation, la solution jaune obtenue est filtrée sur un buchner garni d'une plaque filtrante de cellulose SAM 30 (provenant de la Société LA ROCHETTE SEMPA, FRANCE). Le filtrat obtenu est versé rapidement et sous agitation dans un mélange à 20°C de 100 cm3 d'eau et 100 cm3 d'acide méthanesulfonique. La suspension est filtrée rapidement sur un verre fritté de porosité 3 garni d'un adjuvant de filtration neutre (terre d'infusoires). Le filtrat est abandonné à l'abri de la lumière pendant 2 heures à 20°C et 4 heures à 4°C. On filtre la suspension obtenue, les cristaux blancs recueillis sont lavés par 5 fois 20 cm3 d'acétone, 5 fois 50 cm3 d'éther et séchés sous pression réduite (0,05 mm de mercure ; 6,7 Pa) à 20°C pendant 16 heures. On recueille 5,05 g de méthanesulfonate cristallisé de l'[(amino-2

thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2, isomère syn, forme E.

Spectre de RMN du proton (250 MHz, $CF_3COOD$, δ en ppm, J en Hz) 3,18 (s, 3H, $C\underline{H}_3SO_3H$) ; 3,89 (s, 2H, $-SCH_2-$) ; 4,31 (s, 3H, $-OCH_3$) ; 5,20 (s, 2H, $>NCH_2-$) ; 5,33 (d, J = 5, 1H, H en 6) ; 6,05 (d, J = 5, 1H, H en 7) ; 7,25 et 7,76 (2d, J = 16, 2H, -CH=CH-) ; 7,5 (s, 1H, H du thiazole) ; 9,76 (s, 1H, -CHO).

Spectre de RMN du proton (250 MHz, DMSO $d_6$, δ em ppm, J en Hz)
- forme aldéhyde (75 %) : bandes caractéristiques 9,56 (s, 0,75 H, -CHO) ; 12,80 (s, 0,75 H, -NH- de la triazine).
- forme hydrate (25 %) : bandes caractéristiques 5,03 (t, J = 5, 0,25 H, $-CH_2C\underline{H}(OH)_2$) ; 12,64 (s, 0,25 H, -NH- de la triazine).

Diagramme de diffraction des poudres aux rayons X [rayonnement CuKα avec filtre de nickel, λ = 1,54 Å]

| d (Å) | 4,55 | 3,68 | 3,77 | 6,06 | 7,44 | 3,20 | 8,67 | 3,95 | 5,50 | 5,07 |
|---|---|---|---|---|---|---|---|---|---|---|
| $I/I_1$ | 1 | 0,88 | 0,86 | 0,80 | 0,70 | 0,62 | 0,58 | 0,56 | 0,36 | 0,27 |

Spectre infra-rouge (KBr), bandes caractéristiques ($cm^{-1}$) (formule 6).

| | | | | |
|---|---|---|---|---|
| 3400 F (dont $H_2O$) | 2730 ép | 1360 F | 1000 m | 710 tf |
| 3280 F | 2620 ép | 1310 m | 970 ép | 695 tf |
| 3220 ép | 1770 tF | 1260 m | 950 m | 665 ép |
| 3180 ép | 1720 tF | 1245 ép | 885 ép | 635 m |
| 3120 ép | 1665 tF | 1215 ép | 870 ép | 625 ép |
| 3070 f | 1635 F | 1200 ép | 855 m | 600 tf |
| 3010 ép | 1585 F | 1185 F | 815 ép | 555 m |
| 2970 ép | 1560 f | 1150 F | 800 m | 530 m |
| 2940 tf | 1550 F | 1110 m | 775 m | 490 ép |
| 2895 f | 1470 ép | 1055 ép | 740 m | 455 ép |
| 2830 ép | 1435 ép | 1045 F | 720 tf | 420 tf |
| 2780 ép | 1420 m | | | 395 tf |

EXEMPLE 7

On porte au reflux 70 cm3 d'un mélange préparé à partir de 50 cm3 d'eau, 50 cm3 de méthanol et 5 cm3 d'acide chlorhydrique. On ajoute 2 g de trifluoracétate de 1'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2, isomère syn forme E amorphe. On traite avec 0,1 g de noir décolorant et filtre à chaud. Un produit cristallise en laissant revenir la température à 20°C. On complète la cristallisation en refroidissant pendant 2 heures à 4°C. On sépare les cristaux par filtration et on les lave avec 6 cm3 du mélange solvant de cristallisation puis 5 cm3 d'éther éthylique. On obtient ainsi 1,4 g du chlorhydrate de 1'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2, isomère syn forme E (hémiacétal méthylique) sous forme de cristaux blanc jaune.

Spectre de RMN du proton (250 MHz, $CF_3COOD$, δ en ppm, J en Hz) 3,89 (s, 2H, $-SCH_2-$) ; 4,31 (s, 3H, $-OCH_3$) ; 5,20 (s, 2H, $>NCH_2-$) ; 5,33 (d, J = 5, 1H, H en 6) ; 6,05 (d, J = 5, 1H, H en 7) ; 7,25 et 7,76 (2d, J = 16, 2H, -CH=CH-) ; 7,5 (s, 1H, H du thiazole) ; 9,76 (s, 1H, -CHO).

Spectre de RMN du proton (250 MHz, DMSO $d_6$, δ en ppm, J en Hz)
- hémiacétal méthylique (60 %) : bandes caractéristiques 3,23 (s, 1,8 H, $-CH\langle^{OCH_3}_{OH}$) ; 4,71 (t, J = 5, 0,6 H, $-CH_2-\underline{CH}\langle^{OH}_{OCH_3}$) ; 12,68 (s, 0,6 H, -NH- de la triazine).
- forme aldéhyde (40 %) : bandes caractéristiques 9,56 (s, 0,4 H, -CHO) ; 12,80 (s, 0,1 H, -NH- de la triazine).

Diagramme de diffraction des poudres aux rayons X [rayonnement CuKα avec filtre de nickel, λ = 1,54 Å]

| d (Å) | 7,56 | 3,62 | 3,35 | 5,64 | 8,67 | 3,92 | 3,99 | 3,49 | 3,80 | 3,02 |
|---|---|---|---|---|---|---|---|---|---|---|
| $I/I_1$ | 1 | 1 | 0,96 | 0,89 | 0,84 | 0,84 | 0,80 | 0,80 | 0,71 | 0,69 |

Spectre infra-rouge (KBr), bandes caractéristiques ($cm^{-1}$) (figure 7).

| | | | | |
|---|---|---|---|---|
| 3460 ép (dont $H_2O$) | 2400 ép | 1400 ép | 1055 F | 705 ép |
| 3370 ép | 1765 tF | 1370 F | 995 m | 690 f |
| 3290 F | 1715 tF | 1355 ép | 955 ép | 670 ép |
| 3220 ép | 1670 F | 1340 ép | 940 m | 640 ép |
| 3150 ép | 1660 ép | 1310 ép | 885 ép | 625 m |
| 3065 f | 1630 F | 1260 m | 860 ép | 600 ép |
| 2980 ép | 1590 F | 1220 ép | 850 m | 565 f |
| 2940 ép | 1560 F | 1200 F | 815 ép | 505 tf |
| 2870 ép | 1545 ép | 1180 m | 800 m | 475 ép |
| 2840 ép | 1530 ép | 1160 f | 780 ép | 425 tf |
| 2730 ép | 1465 ép | 1155 ép | 770 f | 395 tf |
| 2680 ép | 1440 m | 1105 m | 740 f | 340 ép |
| 2600 ép | | | | |

EXEMPLE 8

On met en solution à 20°C,30 g de sel de lithium de l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2, isomère syn, forme E dans 750 cm3 d'eau, ajoute ensuite 750 cm3 d'éthanol et filtre. Au filtrat refroidi à 4°C, on ajoute, sous agitation, 300 cm3 d'acide chlorhydrique 4N. Le mélange qui est resté limpide, donne lieu à une précipitation après amorçage. On laisse pendant 3 heures dans un bain de glace, filtre les cristaux formés, les lave par 100 cm3 d'acide chlorhydrique 0,1 N, 200 cm3 d'éthanol et 100 cm3 d'éther éthylique. Après séchage sous pression réduite (0,05 mm de mercure ; 6,7 Pa) à 20°C pendant 16 heures on obtient 30 g de chlorhydrate de l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2, isomère syn, forme E hémiacétal éthylique sous forme de cristaux jaune pâle.

Spectre de RMN du proton (250 MHz, DMSO $d_6$, δ en ppm, J en Hz) - hémiacétal éthylique (65 %) : bandes caractéristiques 1,07 (t, J = 7, 1,95 H, $-CH_2-CH\langle^{OCH_2\underline{CH}_3}_{OH}$) ; 4,79 (t, J = 5, 0,65 H, $-CH_2\underline{CH}(OH)OC_2H_5$) ; 12,66 (s, 0,65 H, -NH- de la triazine).
- forme aldéhyde (35 %) : bandes caractéristiques 9,56 (s, 0,35 H, -CHO) ; 12,80 (s, 0,35 H, -NH- de la triazine).

Spectre infra-rouge (KBr), bandes caractéristiques ($cm^{-1}$) (figure 8).

| | | | | |
|---|---|---|---|---|
| 3360 ép ($H_2O$) | 1720 tF | 1320 ép | 950 ép | 645 ép |
| 3280 F | 1670 ép | 1310 m | 940 m | 635 m |
| 3230 ép | 1660 tF | 1265 m | 885 ép | 610 ép |
| 3150 ép | 1635 F | 1255 ép | 870 ép | 570 ép |
| 3110 ép | 1585 F | 1230 m | 855 m | 560 f |
| 3070 m | 1560 F | 1200 m | 820 ép | 520 tf |
| 2975 f | 1550 ép | 1185 m | 800 m | 510 ép |
| 2940 tf | 1480 ép | 1170 tf | 780 ép | 485 tf |
| 2900 tf | 1450 ép | 1155 m | 775 m | 460 ép |
| 2865 tf | 1440 m | 1110 m | 740 m | 420 tf |
| 2830 ép | 1430 ép | 1055 F | 725 ép | 400 tf |
| 2640 ép | 1375 ép | 1000 m | 700 tf | 370 ép |
| 1770 tF | 1370 F | 970 tf | 675 tf | |

EXEMPLE 9

On met en suspension 15 g de chlorhydrate de l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2, isomère syn forme E, hémiacétal éthylique cristallisé dans 75 cm3 d'acide chlorhydrique 1N et laisse sous agitation pendant 4 heures à 20°C. Le précipité est filtré, lavé par 25 cm3 d'acide chlorhydrique 1N, puis 25 cm3 d'acide chlorhydrique 0,2 N, puis 25 cm3 d'acide chlorhydrique 0,1 N, puis 50 cm3 d'éther éthylique. Les cristaux sont séchés sous pression réduite (0,05 mm de mercure ; 6,7 Pa) pendant 16 heures à 20°C et on obtient

ainsi 13,5 g de chlorhydrate de l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2, isomère syn, forme E sous forme de cristaux jaune pâle.

Spectre de RMN du proton (250 MHz, DMSO $d_6$, δ en ppm, J en Hz)
- forme aldéhyde (60 %) : bandes caractéristiques 9,56 (s, 0,6 H, -CHO) ; 12,80 (s, 0,6 H, -NH- de la triazine).
- forme hydrate (40 %) : bandes caractéristiques 5,03 (t, J = 5, 0,4 H, $-CH_2\underline{CH}(OH)_2$) ; 12,64 (s, 0,4 H, -NH- de la triazine).

Diagramme de diffraction des poudres aux rayons X [rayonnement CuKα avec filtre de nickel, λ = 1,54 Å]

| d (Å) | 11,95 | 8,42 | 3,37 | 4,51 | 3,83 | 6,32 | 5,72 | 3,68 | 6,11 | 4,44 | 3,97 |
|---|---|---|---|---|---|---|---|---|---|---|---|
| $I/I_1$ | 1 | 0,44 | 0,29 | 0,27 | 0,27 | 0,26 | 0,26 | 0,23 | 0,19 | 0,19 | 0,19 |

Spectre infra-rouge (KBr), bandes caractéristiques ($cm^{-1}$) (figure 9).

| | | | | |
|---|---|---|---|---|
| 3360 ép ($H_2O$) | 1770 tF | 1360 F | 1000 m | 715 ép |
| 3290 F | 1710 tF | 1310 m | 965 ép | 695 f |
| 3240 ép | 1680 m | 1260 m | 950 ép | 670 ép |
| 3100 ép | 1660 F | 1235 ép | 945 m | 635 m |
| 3040 ép | 1635 F | 1220 ép | 885 ép | 600 tf |
| 2980 ép | 1590 F | 1200 m | 855 m | 565 tf |
| 2940 f | 1555 F | 1180 m | 815 ép | 510 tf |
| 2900 ép | 1485 ép | 1160 m | 800 m | 480 ép |
| 2750 ép | 1450 ép | 1155 ép | 770 f | 425 tf |
| 2620 ép | 1430 ép | 1105 m | 740 m | 400 f |
| 2450 ép | 1375 ép | 1050 F | 730 ép | |

REVENDICATIONS

1 - Forme cristallisée des sels d'addition avec les acides trifluoracétique, méthanesulfonique ou chlorhydrique de l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2, isomère syn forme E, dont le substituant formylméthyle se trouve à l'état d'aldéhyde libre, d'hydrate d'aldéhyde ou d'hémiacétal ou du mélange de ces formes.

2 - Forme cristallisée de sels d'addition avec les acides trifluoracétique ou méthanesulfonique de l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2 isomère syn forme E selon la revendication 1 dont le radical formylméthyle se présente à l'état de mélange des formes hémiacétal éthylique et aldéhyde libre ou des formes hémiacétal éthylique, hydrate d'aldéhyde et aldéhyde libre, ou encore des formes hydrate d'aldéhyde et aldéhyde libre.

3 - Un procédé de préparation d'un sel cristallisé selon la revendication 1, caractérisé en ce que l'on cristallise en milieu acide le trifluoracétate, le méthanesulfonate ou le chlorhydrate de l'[(amino-2 thiazolyl-4)-2 méthoxyimino-2 acétamido]-7 carboxy-2 [(dioxo-5,6 formylméthyl-4 tétrahydro-1,4,5,6 triazine 1,2,4 yl-3) thio-2 vinyl]-3 oxo-8 thia-5 aza-1 bicyclo [4.2.0] octène-2 isomère syn, forme E, en traitant le produit ci-dessus, un de ses sels ou un solvat formique de ce produit par un excès d'acide trifluoracétique, méthanesulfonique ou chlorhydrique, en milieu aqueux ou hydroorganique.

4 - Un procédé selon la revendication 3, caractérisé en ce que l'on opère en présence d'un excès d'acide compris entre 2 et 300 moles par mole d'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo [4.2.0] octène-2 isomère syn forme E.

5 - Un procédé selon la revendication 3, caractérisé en ce que l'on opère en milieu aqueux ou hydroorganique autre qu'hydroalcoolique et isole le produit cristallisé dont le substituant formylméthyle se trouve à l'état d'aldéhyde libre ou d'hydrate d'aldéhyde ou de leur mélange.

6 - Un procédé selon la revendication 3, caractérisé en ce que l'on opère en milieu hydroalcoolique et isole le produit cristallisé dont le substituant formylméthyle se trouve en totalité à l'état d'hémiacétal ou à l'état de mélange des formes hémiacétal, aldéhyde libre et éventuellement d'hydrate d'aldéhyde.

7 - Un procédé selon l'une des revendications 3 ou 6, caractérisé en ce que l'on opère en milieu hydroalcoolique, l'alcool du milieu étant choisi parmi le méthanol, l'éthanol, le n.propanol ou l'isopropanol.

REVENDICATIONS

1- Un procédé de préparation d'un sel cristallisé d'addition avec les acides trifluoracétique, méthanesulfonique ou chlorhydrique de l'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2, isomère syn forme E, dont le substituant formylméthyle se trouve à l'état d'aldéhyde libre, d'hydrate d'aldéhyde ou d'hémiacétal ou du mélange de ces formes, caractérisé en ce que l'on cristallise en milieu acide le trifluoracétate, le méthanesulfonate ou le chlorhydrate de l'[(amino-2 thiazolyl-4)-2 méthoxyimino-2 acétamido]-7 carboxy-2 [(dioxo-5,6 formylméthyl-4 tétrahydro-1,4,5,6 triazine 1,2,4 yl-3) thio-2 vinyl]-3 oxo-8 thia-5 aza-1 bicyclo [4.2.0] octène-2 isomère syn, forme E, en traitant le produit ci-dessus, un de ses sels ou un solvat formique de ce produit par un excès d'acide trifluoracétique, méthanesulfonique ou chlorhydrique, en milieu aqueux ou hydroorganique.

2 - Un procédé selon la revendication 1, caractérisé en ce que l'on opère en présence d'un excès d'acide compris entre 2 et 300 moles par mole d'[(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo [4.2.0] octène-2 isomère syn forme E.

3 - Un procédé selon la revendication 1, caractérisé en ce que l'on opère en milieu aqueux ou hydroorganique autre qu'hydroalcoolique et isole le produit cristallisé dont le substituant formylméthyle se trouve à l'état d'aldéhyde libre ou d'hydrate d'aldéhyde ou de leur mélange.

4- Un procédé selon la revendication 1, caractérisé en ce que l'on opère en milieu hydroalcoolique et isole le produit cristallisé dont le substituant formylméthyle se trouve en totalité à l'état d'hémiacétal ou à l'état de mélange des formes hémiacétal, aldéhyde libre et éventuellement d'hydrate d'aldéhyde.

5- Un procédé selon l'une des revendications 1 ou 4, caractérisé en ce que l'on opère en milieu hydroalcoolique, l'alcool du milieu étant choisi parmi le méthanol, l'éthanol, le n.propanol ou l'isopropanol.

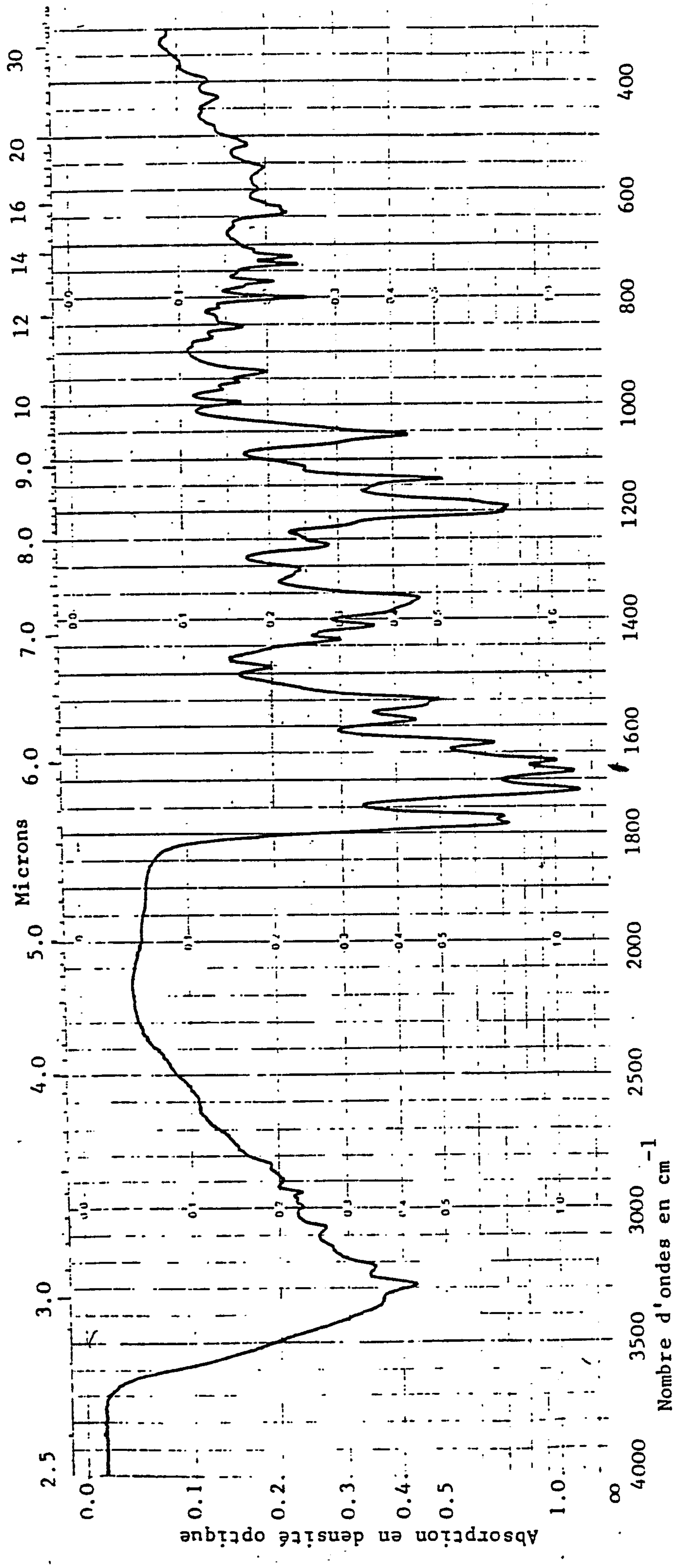
Microns
2.5
3.0
4.0
5.0
6.0
7.0
8.0
9.0
10
12
14
16
20
30
Nombre d'ondes en cm$^{-1}$
4000
3500
3000
2500
2000
1800
1600
1400
1200
1000
800
600
400
Absorption en densité optique
0.0
0.1
0.2
0.3
0.4
0.5
1.0
∞

FIGURE 1

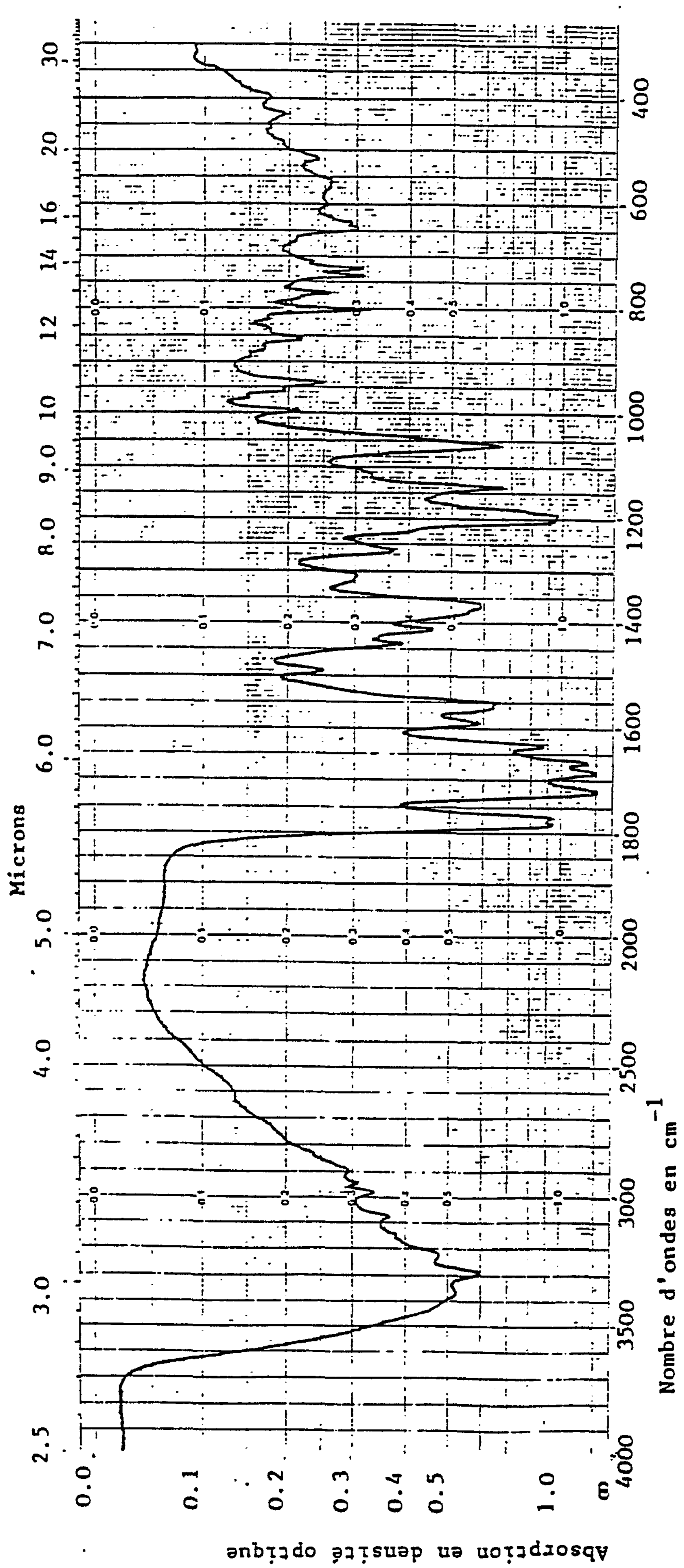
Microns
2.5
3.0
4.0
5.0
6.0
7.0
8.0
9.0
10
12
14
16
20
30
Nombre d'ondes en cm$^{-1}$
4000
3500
3000
2500
2000
1800
1600
1400
1200
1000
800
600
400
Absorption en densité optique
0.0
0.1
0.2
0.3
0.4
0.5
1.0
∞

FIGURE 2

0102896

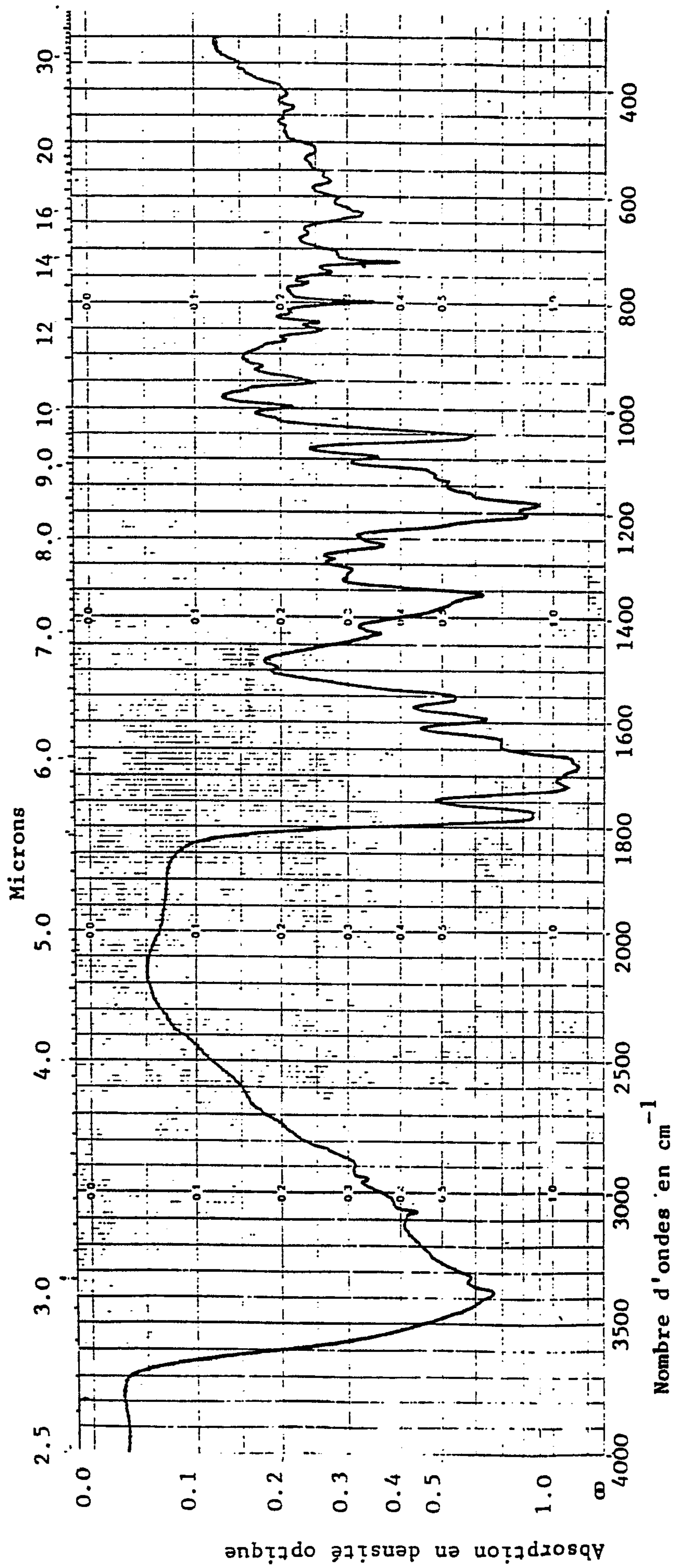


FIGURE 3

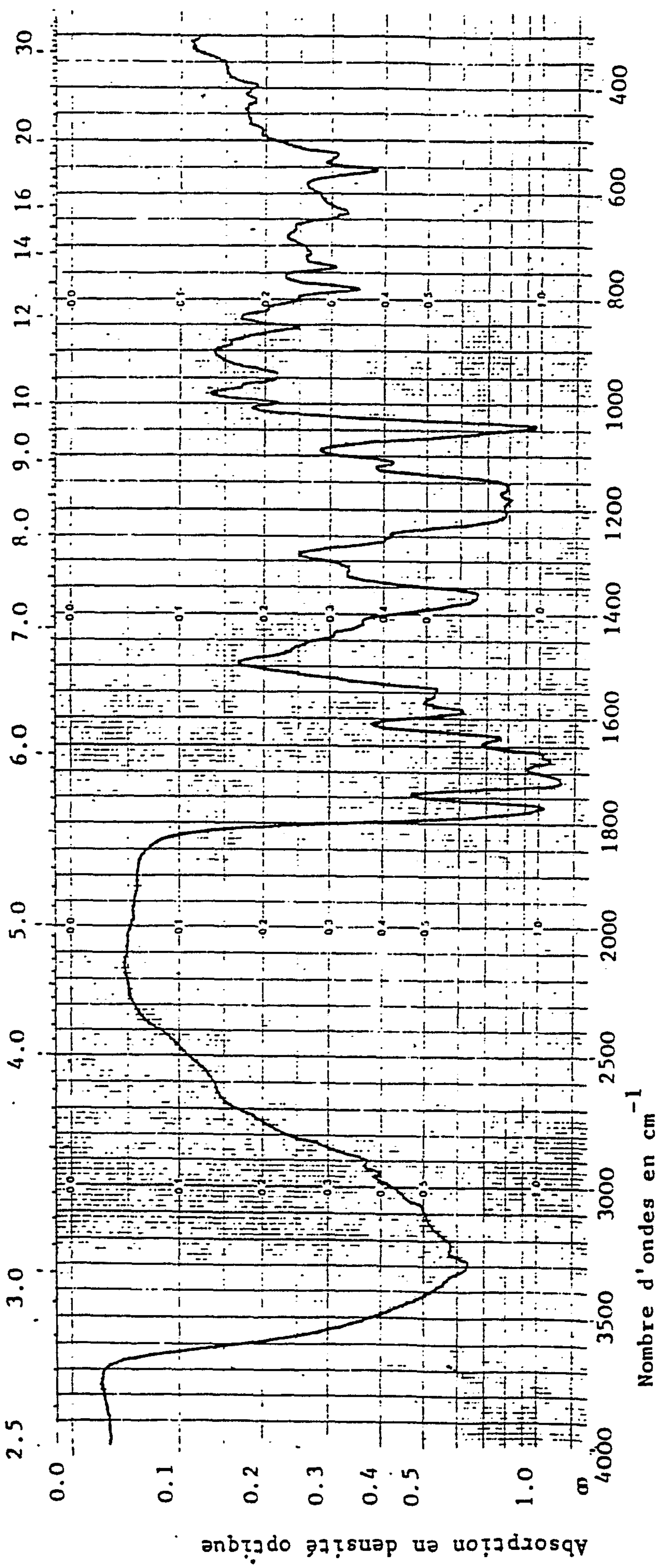
Microns
30
20
16
14
12
10
9.0
8.0
7.0
6.0
5.0
4.0
3.0
2.5
Nombre d'ondes en cm-1
400
600
800
1000
1200
1400
1600
1800
2000
2500
3000
3500
4000
Absorption en densité optique
0.0
0.1
0.2
0.3
0.4
0.5
1.0
∞

FIGURE 4

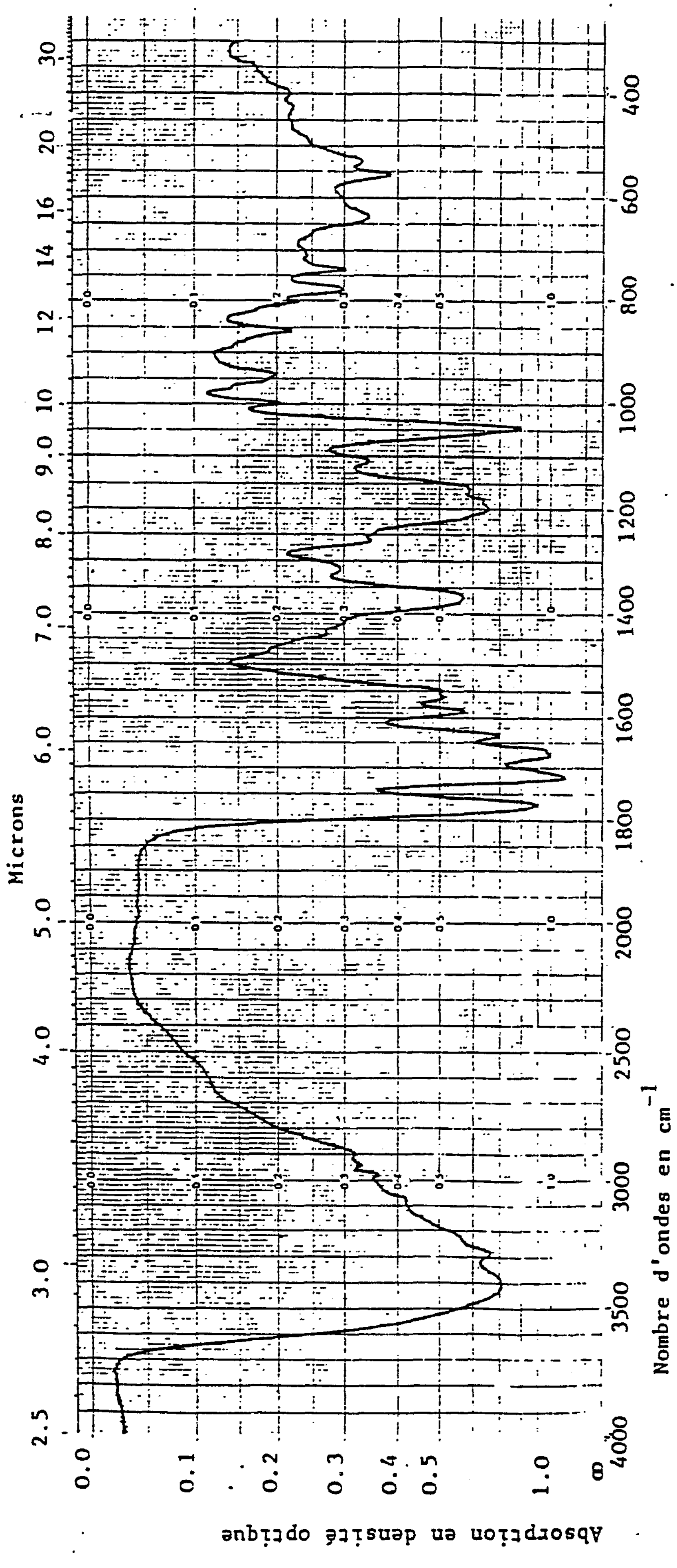
Microns
2.5
3.0
4.0
5.0
6.0
7.0
8.0
9.0
10
12
14
16
20
30
Nombre d'ondes en $cm^{-1}$
4000
3500
3000
2500
2000
1800
1600
1400
1200
1000
800
600
400
Absorption en densité optique
0.0
0.1
0.2
0.3
0.4
0.5
1.0
∞

FIGURE 5

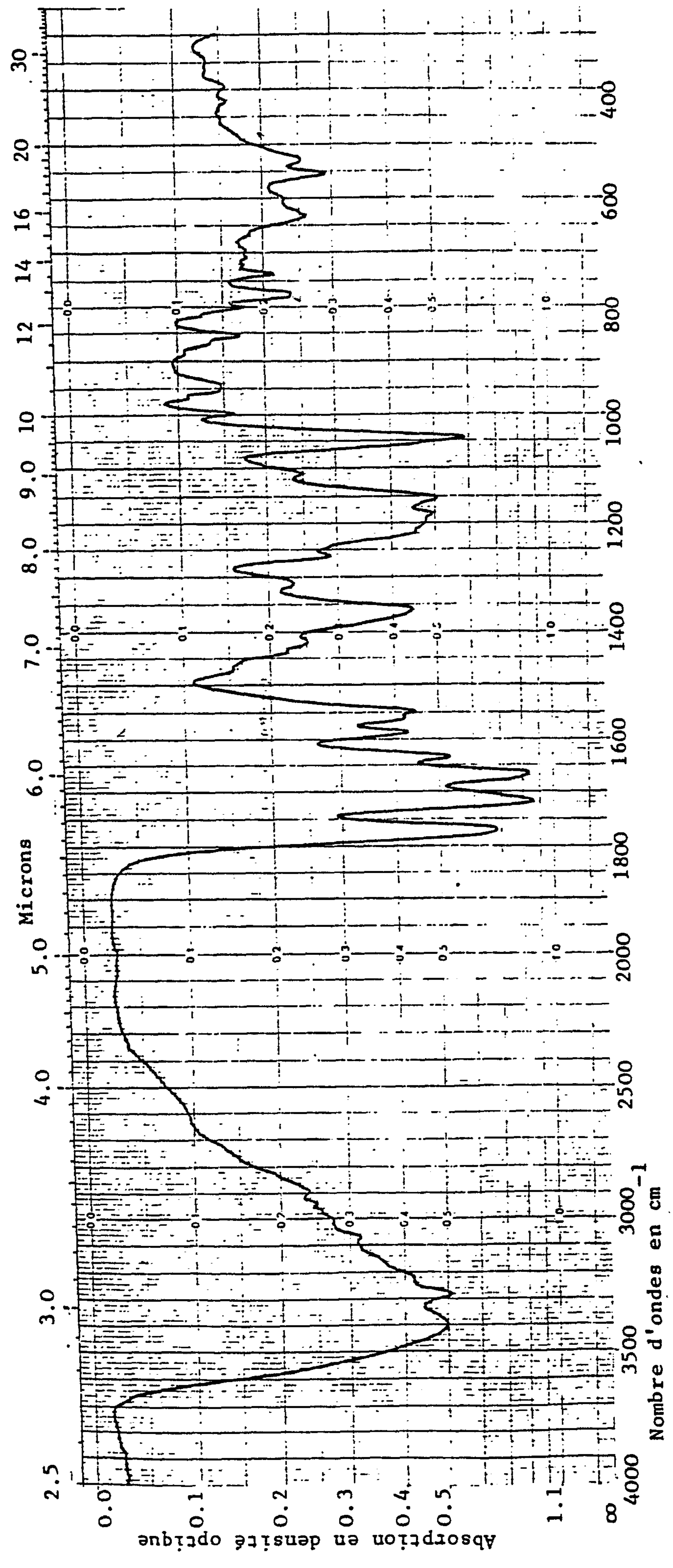
Microns
2.5
3.0
4.0
5.0
6.0
7.0
8.0
9.0
10
12
14
16
20
30
Nombre d'ondes en cm-1
4000
3500
3000
2500
2000
1800
1600
1400
1200
1000
800
600
400
Absorption en densité optique
0.0
0.1
0.2
0.3
0.4
0.5
1.1
∞

FIGURE 6

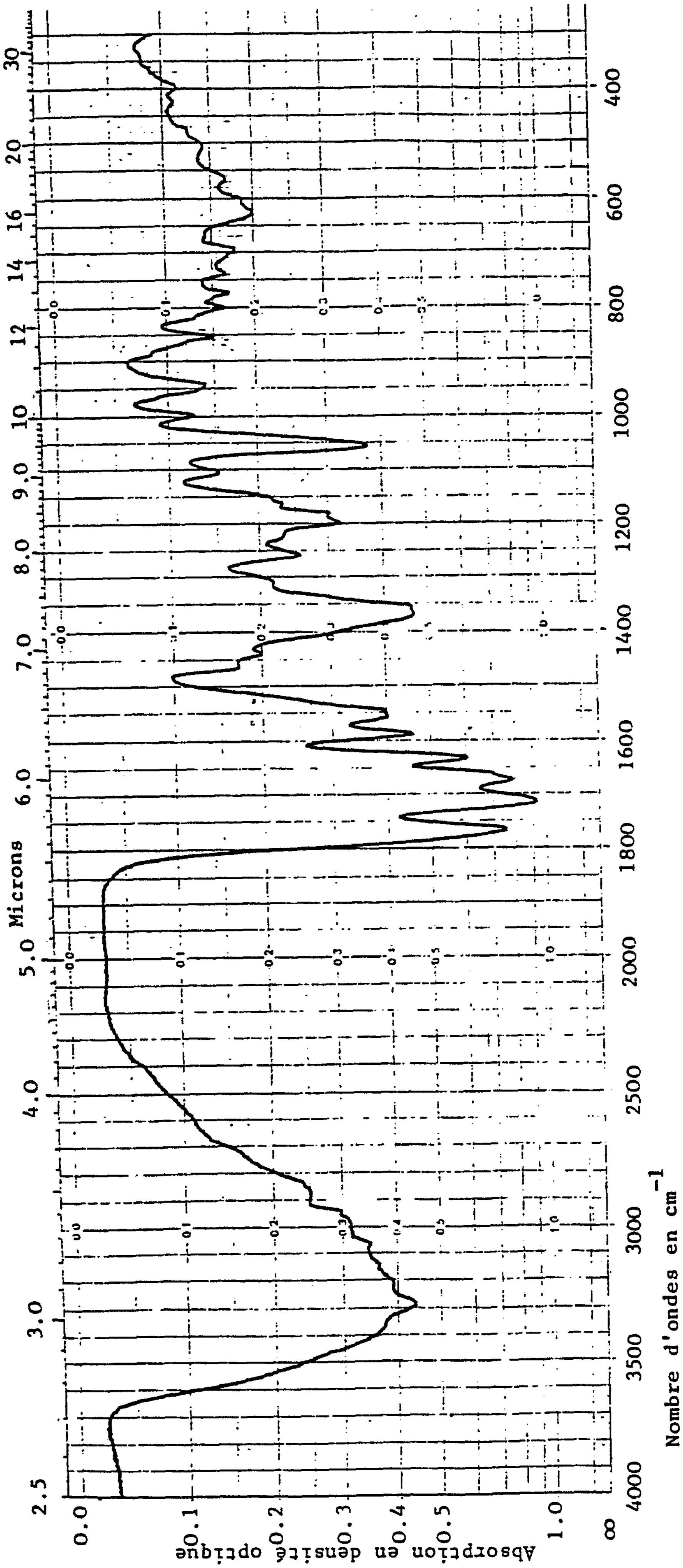
Microns
2.5
3.0
4.0
5.0
6.0
7.0
8.0
9.0
10
12
14
16
20
30
Nombre d'ondes en $cm^{-1}$
4000
3500
3000
2500
2000
1800
1600
1400
1200
1000
800
600
400
Absorption en densité optique
0.0
0.1
0.2
0.3
0.4
0.5
1.0
∞

FIGURE 7

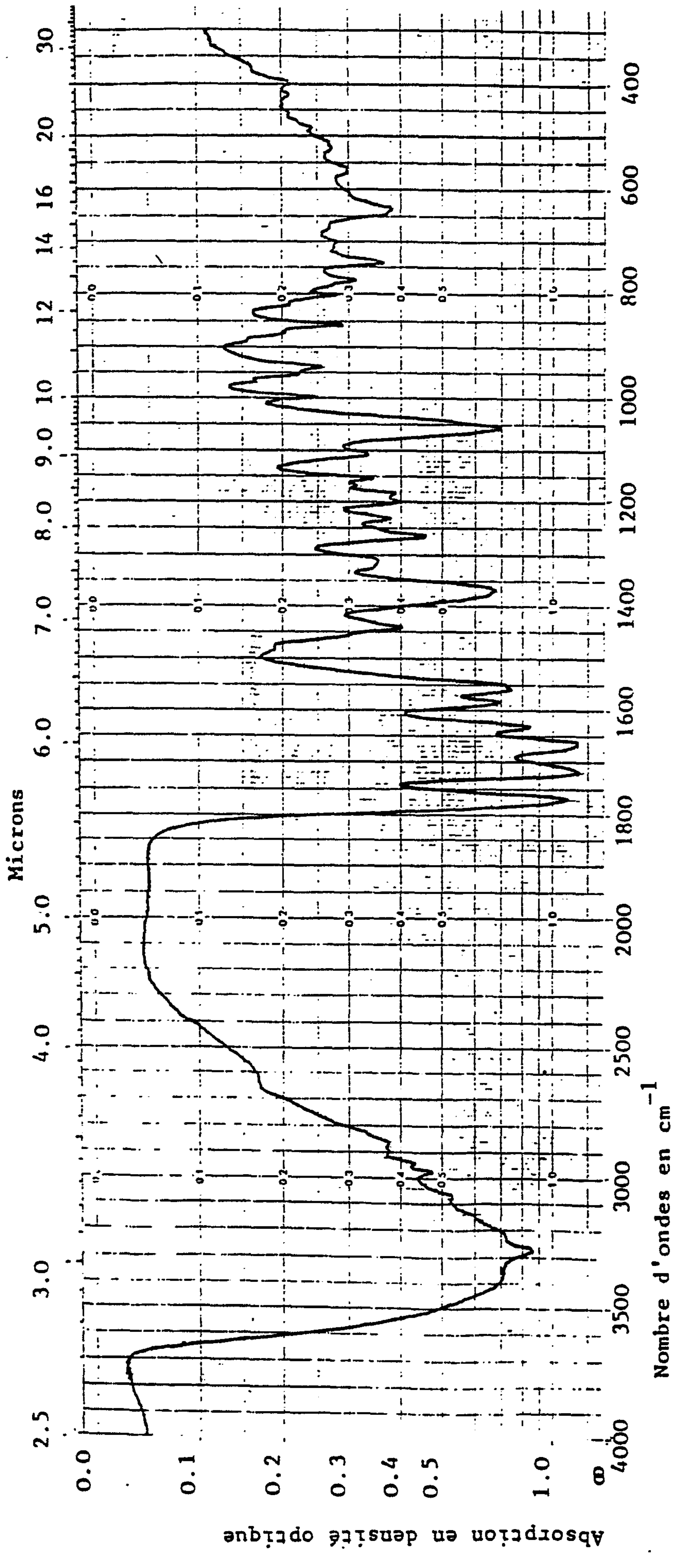

Microns
2.5
3.0
4.0
5.0
6.0
7.0
8.0
9.0
10
12
14
16
20
30
Nombre d'ondes en $cm^{-1}$
4000
3500
3000
2500
2000
1800
1600
1400
1200
1000
800
600
400
Absorption en densité optique
0.0
0.1
0.2
0.3
0.4
0.5
1.0
∞


FIGURE 8

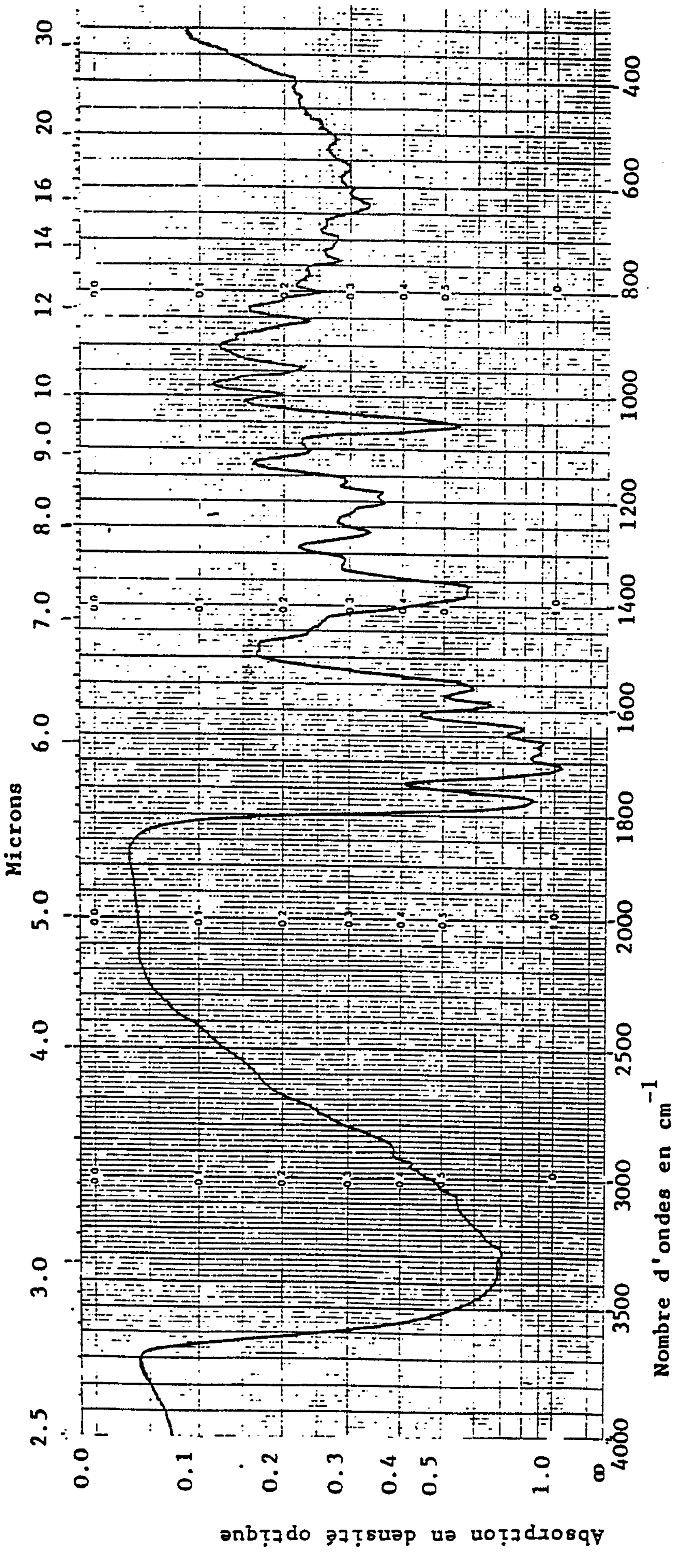
Microns
2.5
3.0
4.0
5.0
6.0
7.0
8.0
9.0
10
12
14
16
20
30
Nombre d'ondes en cm$^{-1}$
4000
3500
3000
2500
2000
1800
1600
1400
1200
1000
800
600
400
Absorption en densité optique
0.0
0.1
0.2
0.3
0.4
0.5
1.0
∞

FIGURE 9

0102896

Office européen des brevets

# RAPPORT DE RECHERCHE EUROPEENNE

Numéro de la demande

EP 83 40 1698

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| D,A | US-A-4 307 116 (DANIEL FARGE et al.)<br>* Colonnes 70-71 *<br>----- | 1 | C 07 D 501/24 |
| | | | DOMAINES TECHNIQUES RECHERCHES (Int. Cl. 3) |
| | | | C 07 D 501/00 |

Le présent rapport de recherche a été établi pour toutes les revendications

| Lieu de la recherche | Date d achèvement de la recherche | Examinateur |
|---|---|---|
| LA HAYE | 22-11-1983 | LUYTEN H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82